# EUROPEAN PATENT APPLICATION

(11) **EP 4 180 540 A1**
(43) Date of publication of application: **17.05.2023**
(21) Application number: 22196985.0
(22) Date of filing: 19.11.2018
(51) Int. Cl.: C12Q 1/6886

(54) **METHOD FOR ASSESSING PROGNOSIS OR RISK STRATIFICATION OF LIVER CANCER BY USING CPG METHYLATION VARIATION IN GENE**

(30) Priority: 22.11.2017 KR 20170156527; 09.11.2018 KR 20180137644
(62) Divisional of application: 18881098.0
(71) Applicant: Lepidyne Co., Ltd., Seongdong-gu Seoul 04778 (KR)
(72) Inventor: KIM, Young Joon, 06024 Gangnam-gu, Seoul (KR); KIM, Da Won, 06366 Gangnam-gu, Seoul (KR); CHOI, Won Young, 04351 Yongsan-gu, Seoul (KR); LEE, Jung Woo, 03722 Seodaemun-gu, Seoul (KR); JUNG, Min Hyeok, 13600 Bundang-gu, Seongnam-si, Gyeonggi-do (KR); HA, Jeong Sil, 06024 Gangnam-gu, Seoul (KR); KIM, Ji Won, 13332 Sujeong-gu, Seongnam-si, Gyeonggi-do (KR); LEE, Yeon Su, 10085 Gimpo-si, Gyeonggi-do (KR); HWANG, Jung Ah, 21378 Bupyeong-gu, Incheon (KR); KIM, Tae You, 06001 Gangnam-gu, Seoul (KR); LIM, Yoo Joo, 06355 Gangnam-gu, Seoul (KR)
(74) Representative: Vossius & Partner Patentanwälte Rechtsanwälte mbB

(57) **Abstract**

The present invention relates to a method for assessing the prognosis or risk stratification of liver cancer by using a clinical specimen mixed with a normal tissue, wherein at least one CpG site that shows a low methylation level in normal and blood tissues but a high methylation level in only a cancer tissue is measured for methylation level.

## Description

### [Technical Field]

The present invention relates to a method of assessing liver cancer-related risk by measuring the extent of methylation at a CpG site of a specific gene.

### [Background Art]

Cancer is a disease in which cell division continues because the cell cycle is not regulated, and grows rapidly as it infiltrates into surrounding tissues and spreads to various regions of the body, thereby threatening life.

Cancer occurring in the liver is called liver cancer, and is one of the most prevalent cancers worldwide. In Korea, the mortality of liver cancer is very high (23 per 100,000 people), and approximately 10% of all deaths are associated with hepatitis, cirrhosis and liver cancer.

Liver cancer may be classified into metastatic liver cancer in which cancer in a different tissue spreads to the liver and hepatocellular carcinoma (HCC) in which cancer occurs in liver cells themselves, and since HCC accounts for 90% of all types of liver cancer, most cases refer to HCC.

Liver cancer is diagnosed by imaging methods such as ultrasound imaging, computed tomography (CT), magnetic resonance imaging (MRI) and hepatic angiography. Ultrasound imaging is highly influenced by sensitivity according to the size of liver cancer, and is used as a primary imaging method for detecting the onset of liver cancer.

Large liver cancer tissues of 5 cm or more show sensitivity of 75% or more, whereas small liver cancer tissues of less than 1 cm show sensitivity of approximately 42% (Gomaa et al., World J Gastro., 15:1301, 2009).

Computed tomography (CT) is an examination tool with the highest sensitivity, and the sensitivity of diagnosis may be almost 100% for liver cancer having a size of 2 cm or more, 93% for that having a size of 1 to 2 cm, and almost 60% for that having a size of 1 cm or less (Gomaa et al., World J Gastro., 15:1301, 2009).

However, this is a burdensome examination tool to be used as a routine screening test for the general public due to a relatively high cost.

In the case of liver cancer, the size of a tumor at the time of diagnosis is associated with prognosis, and to increase a patient's survival rate, liver cancer has to be detected early. Therefore, there is an urgent need for the development of diagnostic technology that can detect liver cancer early with high sensitivity.

Meanwhile, epigenetics is the field of researching the regulation of gene expression which occurs while the base sequence of DNA is not changed. Epigenetics studies the regulation of gene expression through epigenetic mutations such as DNA methylation, miRNA or histone acetylation, methylation, phosphorylation and ubiquitination.

The DNA methylation is the most studied epigenetic mutation. An epigenetic mutation may cause a gene function mutation and a change to tumor cells. Therefore, DNA methylation is associated with the expression (or suppression and induction) of regulatory genes for a disease in cells, and recently, methods for diagnosing cancer by measuring DNA methylation have been suggested.

DNA methylation mainly occurs at cytosine in the CpG island of the promoter site of a specific gene, and thereby, binding of a transcription factor is disturbed to block the expression of a specific gene (gene silencing), which is a main mechanism by which the function of the gene is lost without mutation in a coding sequence.

DNA methylation in a non-translation region such as an enhancer or a regulatory site, in addition to the promoter region of the gene, also works with the structural mutation of a chromosome and histone modification, and is known to become a causative mechanism for various diseases. This abnormal methylation/demethylation in the CpG island was reported in various diseases including cancer, and attempts to use it for diagnosis of various diseases by investigating the promoter methylation of a disease-related gene have been actively made.

The inventors selected a methylation site of a gene related to the onset of liver cancer, and according to an experiment for verifying this, intended to provide a method of diagnosing the risk or prognosis of liver cancer.

Throughout the specification, numerous papers and patent documents are referred and citations thereof are presented. The disclosures of cited papers and patent documents are incorporated herein by reference in their entirety to more clearly explain the level of the technical field to which the present invention belongs and the contents of the present invention.

### [Disclosure]

### [Technical Problem]

To solve the above-described problems of the prior art, the present invention is directed to providing a method of diagnosing the risk or prognosis of liver cancer by measuring a methylation level of a specimen using a specific probe which shows low methylation in normal tissue or blood but shows a high methylation level only in liver cancer tissue to find the risk of liver cancer early.

### [Technical Solution]

According to one aspect of the present invention, a method of assessing the prognosis or risk of liver cancer, which includes: (a) isolating DNA from a biological sample of a subject; and (b) measuring a methylation level of a CpG site at a location selected from the group consisting of the sequence from 25438725 to 25439276 on chromosome #2, the sequence from 95941906 to 95942979 on chromosome #12, the sequence from 134597357 to 134602649 on chromosome #10, the sequence from 144649774 to 144651774 on chromosome #8, the sequence from 47998899 to 47999517 on chromosome #1, the sequence from 26394102 to 26396102 on chromosome #2, the sequence from 104510870 to 104513913 on chromosome #8, the sequence from 98289604 to 98290404 on chromosome #8, the sequence from 63281034 to 63281347 on chromosome #2, the sequence from 67873388 to 67875600 on chromosome #8, the sequence from 76555366 to 76556079 on chromosome #4, the sequence from 63782394 to 63790471 on chromosome #1, the sequence from 7849945 to 7850439 on chromosome #5, the sequence from 39186777 to 39187968 on chromosome #2, and the sequence from 74207665 to 74208665 on chromosome #14 in the isolated DNA, is provided.

In one embodiment, the method may measure a methylation level at two or more CpG sites.

In one embodiment, the sequence from 25438725 to 25439276 on chromosome #2 may have the base sequence of SEQ ID NO: 1, the sequence from 95941906 to 95942979 on chromosome #12 may have the base sequence of SEQ ID NO: 2, the sequence from 134597357 to 134602649 on chromosome #10 may have the base sequence of SEQ ID NO: 3, the sequence from 144649774 to 144651774 on chromosome #8 may have the base sequence of SEQ ID NO: 4, the sequence from 47998899 to 47999517 on chromosome #1 may have the base sequence of SEQ ID NO: 5, the sequence from 26394102 to 26396102 on chromosome #2 may have the base sequence of SEQ ID NO: 6, the sequence from 104510870 to 104513913 on chromosome #8 may have the base sequence of SEQ ID NO: 7, the sequence from 98289604 to 98290404 on chromosome #8 may have the base sequence of SEQ ID NO: 8, the sequence from 63281034 to 63281347 on chromosome #2 may have the base sequence of SEQ ID NO: 9, the sequence from 67873388 to 67875600 on chromosome #8 may have the base sequence of SEQ ID NO: 10, the sequence from 76555366 to 76556079 on chromosome #4 may have the base sequence of SEQ ID NO: 11, the sequence from 63782394 to 63790471 on chromosome #1 may have the base sequence of SEQ ID NO: 12, the sequence from 7849945 to 7850439 on chromosome #5 may have the base sequence of SEQ ID NO: 13, the sequence from 39186777 to 39187968 on chromosome #2 may have the base sequence of SEQ ID NO: 14, and the sequence from 74207665 to 74208665 on chromosome #14 may have the base sequence of SEQ ID NO: 15.

In one embodiment, a CpG site of the sequence from 25438725 to 25439276 on chromosome #2 may be located at 25439110 of chromosome #2, a CpG site of the sequence from 95941906 to 95942979 on chromosome #12 may be located at 95941988 of chromosome #12, a CpG site of the sequence from 134597357 to 134602649 on chromosome #10 may be located at 134599823 of chromosome #10, a CpG site of the sequence from 144649774 to 144651774 on chromosome #8 may be located at 144651002 of chromosome #8, a CpG site of the sequence from 47998899 to 47999517 on chromosome #1 may be located at 47999163 of chromosome #1, a CpG site of the sequence from 26394102 to 26396102 on chromosome #2 may be located at 26395458 of chromosome #2, a CpG site of the sequence from 104510870 to 104513913 on chromosome #8 may be located at 104512877 of chromosome #8, a CpG site of the sequence from 98289604 to 98290404 on chromosome #8 may be located at 98290148 of chromosome #8, a CpG site of the sequence from 63281034 to 63281347 on chromosome #2 may be located at 63281139 of chromosome #2, a CpG site of the sequence from 67873388 to 67875600 on chromosome #8 may be located at 67874178 of chromosome #8, a CpG site of the sequence from 76555366 to 76556079 on chromosome #4 may be located at 76555832 of chromosome #4, a CpG site of the sequence from 63782394 to 63790471 on chromosome #1 may be located at 63789278 of chromosome #1, a CpG site of the sequence from 7849945 to 7850439 on chromosome #5 may be located at 7850070 of chromosome #5, a CpG site of the sequence from 39186777 to 39187968 on chromosome #2 may be located at 39187533 of chromosome #2, and a CpG site of the sequence from 74207665 to 74208665 on chromosome #14 may be located at 74208165 of chromosome #14.

In one embodiment, the biological sample may be one selected from the group consisting of tissue, cells, blood, plasma, stool and urine derived from a patient with suspected liver cancer or a subject diagnosed with liver cancer.

In one embodiment, step (b) may be performed by one method selected from the group consisting of PCR, methylation-specific PCR, real-time methylation-specific PCR, MethyLight PCR, MethyLight digital PCR, EpiTYPER, PCR using methylated DNA-specific binding protein, quantitative PCR, DNA chip assay, pyrosequencing and bisulfite sequencing.

In one embodiment, the method may further include (c) comparing the methylation level with a methylation level in a normal control.

According to another aspect of the present invention, a kit for diagnosing a risk of the onset of liver cancer, which includes a probe binding to a CpG site at a location selected from the group consisting of the sequence from 25438725 to 25439276 on chromosome #2, the sequence from 95941906 to 95942979 on chromosome #12, the sequence from 134597357 to 134602649 on chromosome #10, the sequence from 144649774 to 144651774 on chromosome #8, the sequence from 47998899 to 47999517 on chromosome #1, the sequence from 26394102 to 26396102 on chromosome #2, the sequence from 104510870 to 104513913 on chromosome #8, the sequence from 98289604 to 98290404 on chromosome #8, the sequence from 63281034 to 63281347 on chromosome #2, the sequence from 67873388 to 67875600 on chromosome #8, the sequence from 76555366 to 76556079 on chromosome #4, the sequence from 63782394 to 63790471 on chromosome #1, the sequence from 7849945 to 7850439 on chromosome #5, the sequence from 39186777 to 39187968 on chromosome #2, and the sequence from 74207665 to 74208665 on chromosome #14, is provided.

In one embodiment, the diagnostic kit may include two or more probes binding to CpG sites.

### [Advantageous Effects]

According to one aspect of the present invention, the possibility of the onset of liver cancer can be effectively predicted using a clinical specimen in which normal tissue is mixed by measuring methylation of a specific CpG site showing a different methylation level from most normal cells including blood as well as cancer and normal tissues.

It should be understood that the effect of the present invention is not limited to the above-described effects, and includes all effects that can be deduced from the configuration of the present invention described in the detailed description or claims of the present invention.

### [Description of Drawings]

FIG. 1 illustrates a liver cancer diagnostic marker selection pipeline of the present invention.

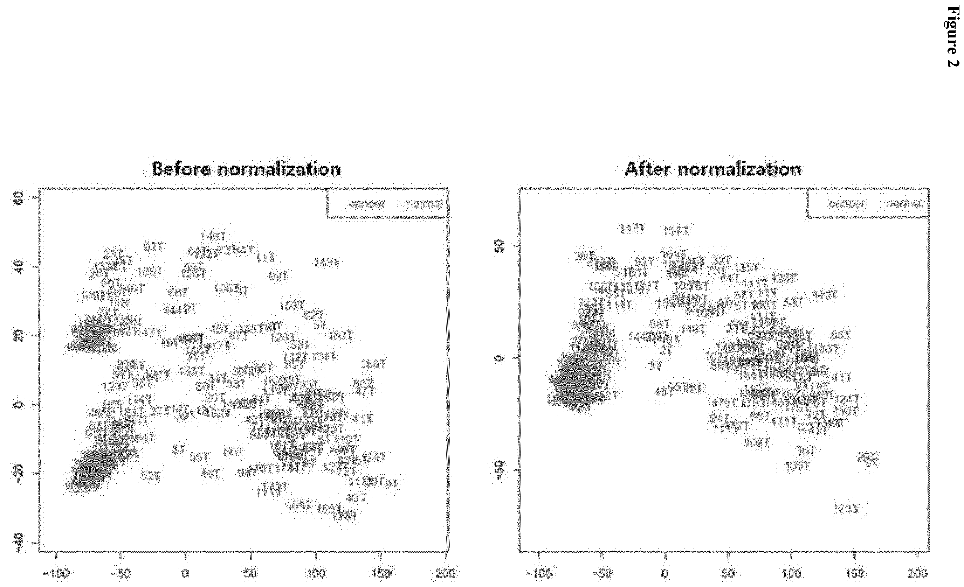

FIG. 2 is a set of graphs showing the distribution of liver cancer patients before (left) and after (right) normalization of DNA methylation data according to an exemplary embodiment of the present invention, respectively.
FIG. 3 is a heat map of differentially methylated probes (DMPs) which are hypermethylated in a liver cancer patient and hypomethylated in a normal person according to an exemplary embodiment of the present invention.
FIG. 4 is a set of heat maps showing the extent of methylation in a liver cancer sample, a normal liver sample and a blood sample for probes selected by heat maps. A red color indicates hypermethylation.
FIG. 5 shows the result of selecting a diagnostic marker according to an exemplary embodiment of the present invention through machine learning.
FIG. 6 is a set of heat maps confirming the extent of methylation of a diagnostic marker according to an exemplary embodiment of the present invention selected through machine learning in a liver cancer sample, a normal liver sample and a blood sample.
FIG. 7 shows a result of evaluating liver cancer diagnostic efficiency of a single probe according to one exemplary embodiment of the present invention. The liver cancer diagnostic efficiency per probe is represented as AUC.

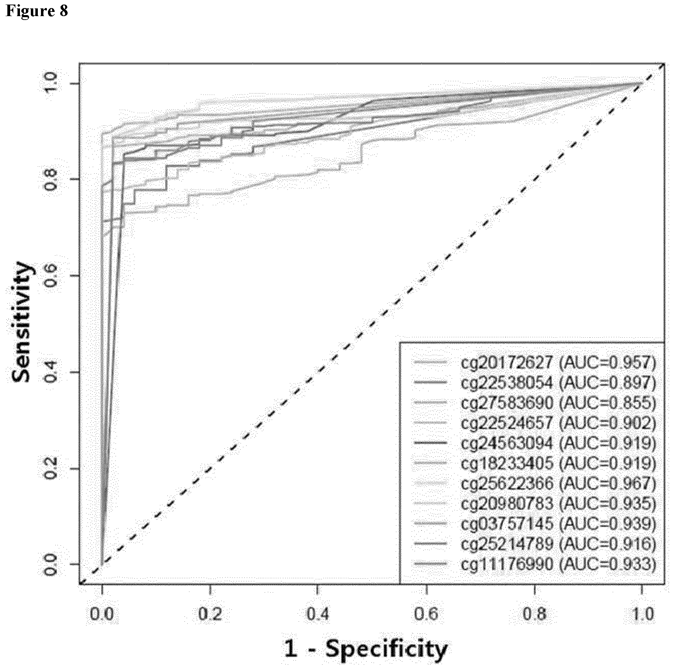

FIG. 8 shows a result of evaluating liver cancer diagnostic efficiency of a single probe according to an exemplary embodiment of the present invention in liver cancer data of the Cancer Genome Atlas (TCGA), which is a public DB. The liver cancer diagnostic efficiency per probe is represented as AUC.
FIG. 9 shows a result of confirming diagnostic efficiency according to the combination of probes (15 kinds) according to an exemplary embodiment of the present invention.
FIG. 10 is a set of heat maps showing the extent of methylation of probes selected according to an exemplary embodiment of the present invention through pyrosequencing. The x-axis represents 196 liver cancer samples in an independent cohort and normal liver samples corresponding thereto, and the y-axis represents CpG sites of a probe (yellow box) and near the probe.
FIG. 11 is a set of heat maps showing the extent of methylation of probes selected according to an exemplary embodiment of the present invention through an EpiTYPER experiment. The x-axis represents 184 liver cancer samples in an independent cohort and normal liver samples corresponding thereto, and the y-axis represents CpG sites of a probe (yellow box) and near the probe.

### [Modes of the Invention]

Hereinafter, the present invention will be described in detail with reference to the accompanying drawings. However, the present invention may be implemented in a variety of different forms, and is not limited to the embodiments described herein.

When one part "includes" a component, it means that, unless particularly stated otherwise, the part may further include another component, rather than excluding it.

Unless defined otherwise, the present invention may be carried out by conventional techniques frequently used in molecular biology, microbiology, protein purification, protein engineering, DNA sequencing, and the recombinant DNA field within the scope of those of ordinary skill in the art. The techniques are known to those of ordinary skill in the art, and described in many standardized textbooks and references.

Unless particularly defined otherwise, all technical and scientific terms used herein have the same meaning as generally understood by those of ordinary skill in the art.

Various scientific dictionaries, including the terms included herein, are well known and available in the art. Although any method and material which is similar or equivalent to those described herein are found in the execution or testing of the present invention, some methods and materials will be described. Depending on the context used by those of skill in the art, various methods and materials can be used, and thus the present invention is not limited to specific methodologies, protocols and reagents.

As used herein, singular forms include plural forms unless specifically stated otherwise. In addition, unless indicated otherwise, nucleic acids are written from left to right in the 5' to 3' direction, respectively, and the sequence of amino acids is written from left to right, in an amino to carboxyl direction. Hereinafter, the present invention will be described in further detail.

According to an aspect of the present invention, a method of assessing the prognosis or risk of liver cancer, which includes measuring a methylation level of one or more CpG sites, is provided.

The term "subject" may be a diagnostic target, which is a human, and the biological sample is a sample isolated from the subject to evaluate the risk of a liver cancer-related disease, including tissue, cells, blood, plasma, peritoneal fluid, synovial fluid, saliva, urine and stool, but the present invention is not limited thereto. Preferably, the biological sample may be blood, and specifically, plasma separated from blood.

In addition, the prognosis or risk of liver cancer may be diagnosed by individually analyzing a methylation level of the CpG site, but the accuracy of diagnosis is preferably enhanced by simultaneously analyzing two, three or four or more CpG sites.

The diagnosis is for determining the susceptibility of a subject for a specific disease or disorder, and preferably, determining whether a subject currently has liver cancer or determining the prognosis of a subject with liver cancer, and may include therametrics.

The "methylation" refers to attachment of a methyl group to a base constituting DNA. Preferably, methylation refers to methylation occurring at cytosine of a specific CpG site of a specific gene.

The "methylated state" refers to the presence or absence of 5-methyl cytosine of one or more CpG dinucleotides in a DNA base sequence. The "methylation level" refers to, for example, a level of methylation present in the DNA base sequences of target DNA-methylated genes in all genomic regions and some non-genomic regions.

The methylation level may be measured by one method selected from the group consisting of PCR, methylation-specific PCR, real-time methylation-specific PCR, MethyLight PCR, MethyLight digital PCR, EpiTYPER, PCR using a methylated DNA-specific binding protein, quantitative PCR, a DNA chip assay, pyrosequencing and bisulfite sequencing, but the present invention is not limited thereto.

The extent of methylation may be identified by a microarray. The microarray may be performed using a probe immobilized on a solid surface. The probe may include a sequence complementary to a sequence of 10 to 100 consecutive nucleotides in each gene containing an SNP.

The CpG site refers to a CpG site present in DNA of the gene. DNA of the gene is the concept including a series of all structural units that are required for expression and operably linked to each other, and includes, for example, a promoter region, a protein coding region (open reading frame, ORF) and a terminator region.

Therefore, the CpG site of the gene may be present in the promoter region, protein coding region (OFR) or terminator region of a corresponding gene. As a preferable example, the CpG site of the gene may be a CpG site present in the promoter region of the gene.

The CpG site may be present in one or more base sequences selected from the group consisting of the sequence from 25438725 to 25439276 on chromosome #2, the sequence from 95941906 to 95942979 on chromosome #12, the sequence from 134597357 to 134602649 on chromosome #10, the sequence from 144649774 to 144651774 on chromosome #8, the sequence from 47998899 to 47999517 on chromosome #1, the sequence from 26394102 to 26396102 on chromosome #2, the sequence from 104510870 to 104513913 on chromosome #8, the sequence from 98289604 to 98290404 on chromosome #8, the sequence from 63281034 to 63281347 on chromosome #2, the sequence from 67873388 to 67875600 on chromosome #8, the sequence from 76555366 to 76556079 on chromosome #4, the sequence from 63782394 to 63790471 on chromosome #1, the sequence from 7849945 to 7850439 on chromosome #5, the sequence from 39186777 to 39187968 on chromosome #2, and the sequence from 74207665 to 74208665 on chromosome #14.

The sequence from 25438725 to 25439276 on chromosome #2 may have the base sequence of SEQ ID NO: 1, the sequence from 95941906 to 95942979 on chromosome #12 may have the base sequence of SEQ ID NO: 2, the sequence from 134597357 to 134602649 on chromosome #10 may have the base sequence of SEQ ID NO: 3, the sequence from 144649774 to 144651774 on chromosome #8 may have the base sequence of SEQ ID NO: 4, the sequence from 47998899 to 47999517 on chromosome #1 may have the base sequence of SEQ ID NO: 5, the sequence from 26394102 to 26396102 on chromosome #2 may have the base sequence of SEQ ID NO: 6, the sequence from 104510870 to 104513913 on chromosome #8 may have the base sequence of SEQ ID NO: 7, the sequence from 98289604 to 98290404 on chromosome #8 may have the base sequence of SEQ ID NO: 8, the sequence from 63281034 to 63281347 on chromosome #2 may have the base sequence of SEQ ID NO: 9, the sequence from 67873388 to 67875600 on chromosome #8 may have the base sequence of SEQ ID NO: 10, the sequence from 76555366 to 76556079 on chromosome #4 may have the base sequence of SEQ ID NO: 11, the sequence from 63782394 to 63790471 on chromosome #1 may have the base sequence of SEQ ID NO: 12, the sequence from 7849945 to 7850439 on chromosome #5 may have the base sequence of SEQ ID NO: 13, the sequence from 39186777 to 39187968 on chromosome #2 may have the base sequence of SEQ ID NO: 14, and the sequence from 74207665 to 74208665 on chromosome #14 may have the base sequence of SEQ ID NO: 15.

The CpG site of the sequence from 25438725 to 25439276 on chromosome #2 may be located at 25439110 of chromosome #2, the CpG site of the sequence from 95941906 to 95942979 on chromosome #12 may be located at 95941988 of chromosome #12, the CpG site of the sequence from 134597357 to 134602649 on chromosome #10 may be located at 134599823 of chromosome #10, the CpG site of the sequence from 144649774 to 144651774 on chromosome #8 may be located at 144651002 of chromosome #8, the CpG site of the sequence from 47998899 to 47999517 on chromosome #1 may be located at 47999163 of chromosome #1, the CpG site of the sequence from 26394102 to 26396102 on chromosome #2 may be located at 26395458 of chromosome #2, the CpG site of the sequence from 104510870 to 104513913 on chromosome #8 may be located at 104512877 of chromosome #8, the CpG site of the sequence from 98289604 to 98290404 on chromosome #8 may be located at 98290148 of chromosome #8, the CpG site of the sequence from 63281034 to 63281347 on chromosome #2 may be located at 63281139 of chromosome #2, the CpG site of the sequence from 67873388 to 67875600 on chromosome #8 may be located at 67874178 of chromosome #8, the CpG site of the sequence from 76555366 to 76556079 on chromosome #4 may be located at 76555832 of chromosome #4, the CpG site of the sequence from 63782394 to 63790471 on chromosome #1 may be located at 63789278 of chromosome #1, the CpG site of the sequence from 7849945 to 7850439 on chromosome #5 may be located at 7850070 of chromosome #5, the CpG site of the sequence from 39186777 to 39187968 on chromosome #2 may be located at 39187533 of chromosome #2, and the CpG site of the sequence from 74207665 to 74208665 on chromosome #14 may be located at 74208165 of chromosome #14.

According to another aspect of the present invention, a kit for diagnosing a risk of the onset of liver cancer, which includes a probe binding to a CpG site at a location selected from the group consisting of the sequence from 25438725 to 25439276 on chromosome #2, the sequence from 95941906 to 95942979 on chromosome #12, the sequence from 134597357 to 134602649 on chromosome #10, the sequence from 144649774 to 144651774 on chromosome #8, the sequence from 47998899 to 47999517 on chromosome #1, the sequence from 26394102 to 26396102 on chromosome #2, the sequence from 104510870 to 104513913 on chromosome #8, the sequence from 98289604 to 98290404 on chromosome #8, the sequence from 63281034 to 63281347 on chromosome #2, the sequence from 67873388 to 67875600 on chromosome #8, the sequence from 76555366 to 76556079 on chromosome #4, the sequence from 63782394 to 63790471 on chromosome #1, the sequence from 7849945 to 7850439 on chromosome #5, the sequence from 39186777 to 39187968 on chromosome #2, and the sequence from 74207665 to 74208665 on chromosome #14, is provided.

The probe may be used as a hybridizable array element and immobilized on a substrate.

The substrate may be a suitable rigid or semi-rigid support, and include, for example, a membrane, a filter, a chip, a slide, a wafer, a fiber, a magnetic or nonmagnetic bead, a gel, tubing, a plate, a polymer, a microparticle, and a capillary tube. The hybridizable array element may be arranged on the substrate and immobilized thereon.

The immobilization may be performed by a chemical binding method or a covalent bonding method such as UV. For example, the hybridizable array element may be bound to a glass surface modified to include an epoxy compound or an aldehyde group, and bound to a polylysine-coated surface by UV. In addition, the hybridizable array element may be bound to the substrate by linkers (e.g., an ethylene glycol oligomer and a diamine).

Sample DNA applied to the microarray may be labeled, and hybridized with an array element. Hybridization conditions may be changed in various ways, and the detection and analysis of the extent of hybridization may be carried out in various ways according to a labeling substance.

Labeling of the probe may provide a signal that allows the detection of hybridization, and may be linked to an oligonucleotide.

The label may include a fluorophore (e.g., fluorescein, phycoerythrin, rhodamine, lissamine, Cy3 or Cy5 (Pharmacia)), a chromophore, a chemiluminophore, a magnetic particle, a radioisotope (P32 or S35), a mass label, an electron dense particle, an enzyme (alkaline phosphatase or horseradish peroxidase), a cofactor, a substrate for an enzyme, a heavy metal (e.g., gold), an antibody, streptavidin, biotin, digoxigenin, or a hapten with a specific binding partner such as a chelating group, but the present invention is not limited thereto.

The label may be labeled by various methods conventionally performed in the art, for example, a nick translation method, a random priming method (Multiprime DNA labelling systems booklet, "Amersham" (1989)) and a kination method (Maxam & Gilbert, Methods in Enzymology, 65:499(1986)).

The label may provide a signal that can be detected by fluorescence, radioactivity, colorimetry, gravimetry, X-ray diffraction or absorption, magnetism, enzymatic activity, mass spectrometry, binding affinity, hybridization radiofrequency or nanocrystals.

The nucleic acid sample to be analyzed may be prepared using mRNA obtained from various biosamples. Instead of the probe, cDNA to be analyzed may be labeled to perform hybridization-based analysis.

When the probe is used, the probe may be hybridized with a cDNA molecule. The suitable hybridization conditions may be determined in a series of steps by an optimization procedure. The procedure may be performed in a series of steps by those of ordinary skill in the art to establish a protocol to be used in a laboratory.

For example, conditions such as a temperature, the concentration of a component, hybridization and washing time, components of a buffer solution, pH and ionic strength depend on various parameters such as a probe length, a GC content and a target nucleotide sequence. For detailed conditions for the hybridization, Joseph Sambrook et al., Molecular Cloning, A Laboratory Manual, Cold Spring Harbor Laboratory Press, Cold Spring Harbor, N.Y. (2001); and M.L.M. Anderson, Nucleic Acid Hybridization, Springer-Verlag New York Inc. N.Y. (1999) may be referenced.

For example, high stringent conditions among the stringent conditions may refer to hybridization under conditions of 0.5 M NaHPO₄, 7% sodium dodecyl sulfate (SDS), and 1 mM EDTA at 65 °C, and washing in 0.1 × standard saline citrate (SSC)/0.1% SDS at 68 °C. Alternatively, the high stringent condition may refer to washing in 6x SSC/0.05% sodium pyrophosphate at 48 °C, and a low stringent condition may refer to washing in 0.2 x SSC/0.1% SDS at 42 °C.

After the hybridization, a hybridization signal emitted by hybridization may be detected. For example, when the probe is labeled with an enzyme, hybridization may be confirmed by reacting a substrate of the enzyme with a hybridization reaction product.

The enzyme and substrate may be a peroxidase (e.g., horseradish peroxidase) and chloronaphthol, aminoethyl carbazole, diaminobenzidine, D-luciferin, bis-N-methylacridinium nitrate (Lucigenin), resorufin benzyl ether, luminol, the Amplex Red reagent (10-acetyl-3,7-dihydroxyphenoxazine), HYR (p-phenylenediamine-HCl and pyrocatechol), tetramethylbenzidine (TMB), 2,2'-azine-di[3-ethylbenzthiazoline sulfonate] (ABTS), o-phenylenediamine (OPD) or naphthol/pyronine; alkaline phosphatase and bromochloroindolyl phosphate (BCIP), nitroblue tetrazolium (NBT) or naphthol-AS-B1-phosphate and an ECF substrate; and glucose oxidase and nitroblue tetrazolium (t-NBT) or phenazine methosulfate (m-PMS).

When the probe is labeled with gold particles, it may be detected by a silver staining method using silver nitrate.

The method of assessing the prognosis or risk of liver cancer may be assessing the possibility of diagnosing liver cancer by various statistical methods. In one embodiment, a statistical method may be a machine learning method, and Maxwell W. Libbrecht, 2015, Nature Reviews Genetics 16: 321-332 may be referenced.

The machine learning is a field of artificial intelligence that has evolved from the study of pattern recognition and computer learning theory. Machine learning is technology of studying and establishing a system for experimental data-based learning, prediction and enhancement of performance by itself and algorithms therefor. The algorithms of machine learning are methods of constructing a specific model to elicit predictions or determinations based on input data, rather than carrying out strictly determined static program commands.

Furthermore, the present invention relates to the following items:

### [ITEMS]

### [Item 1]

A method of assessing the prognosis or risk of liver cancer, comprising:
(a) isolating DNA from a biological sample of a subject; and
(b) measuring a methylation level of a CpG site at a location selected from the group consisting of the sequence from 25438725 to 25439276 on chromosome #2, the sequence from 95941906 to 95942979 on chromosome #12, the sequence from 134597357 to 134602649 on chromosome #10, the sequence from 144649774 to 144651774 on chromosome #8, the sequence from 47998899 to 47999517 on chromosome #1, the sequence from 26394102 to 26396102 on chromosome #2, the sequence from 104510870 to 104513913 on chromosome #8, the sequence from 98289604 to 98290404 on chromosome #8, the sequence from 63281034 to 63281347 on chromosome #2, the sequence from 67873388 to 67875600 on chromosome #8, the sequence from 76555366 to 76556079 on chromosome #4, the sequence from 63782394 to 63790471 on chromosome #1, the sequence from 7849945 to 7850439 on chromosome #5, the sequence from 39186777 to 39187968 on chromosome #2, and the sequence from 74207665 to 74208665 on chromosome #14 in the isolated DNA.

### [Item 2]

The method of item 1, wherein levels of methylation at 2 or more CpG sites are measured.

### [Item 3]

The method of item 1, wherein the sequence from 25438725 to 25439276 on chromosome #2 has the base sequence of SEQ ID NO: 1, the sequence from 95941906 to 95942979 on chromosome #12 has the base sequence of SEQ ID NO: 2, the sequence from 134597357 to 134602649 on chromosome #10 has the base sequence of SEQ ID NO: 3, the sequence from 144649774 to 144651774 on chromosome #8 has the base sequence of SEQ ID NO: 4, the sequence from 47998899 to 47999517 on chromosome #1 has the base sequence of SEQ ID NO: 5, the sequence from 26394102 to 26396102 on chromosome #2 has the base sequence of SEQ ID NO: 6, the sequence from 104510870 to 104513913 on chromosome #8 has the base sequence of SEQ ID NO: 7, the sequence from 98289604 to 98290404 on chromosome #8 has the base sequence of SEQ ID NO: 8, the sequence from 63281034 to 63281347 on chromosome #2 has the base sequence of SEQ ID NO: 9, the sequence from 67873388 to 67875600 on chromosome #8 has the base sequence of SEQ ID NO: 10, the sequence from 76555366 to 76556079 on chromosome #4 has the base sequence of SEQ ID NO: 11, the sequence from 63782394 to 63790471 on chromosome #1 has the base sequence of SEQ ID NO: 12, the sequence from 7849945 to 7850439 on chromosome #5 has the base sequence of SEQ ID NO: 13, the sequence from 39186777 to 39187968 on chromosome #2 has the base sequence of SEQ ID NO: 14, and the sequence from 74207665 to 74208665 on chromosome #14 has the base sequence of SEQ ID NO: 15.

### [Item 4]

The method of item 1, wherein a CpG site of the sequence from 25438725 to 25439276 on chromosome #2 is located at 25439110 of chromosome #2, a CpG site of the sequence from 95941906 to 95942979 on chromosome #12 is located at 95941988 of chromosome #12, a CpG site of the sequence from 134597357 to 134602649 on chromosome #10 is located at 134599823 of chromosome #10, a CpG site of the sequence from 144649774 to 144651774 on chromosome #8 is located at 144651002 of chromosome #8, a CpG site of the sequence from 47998899 to 47999517 on chromosome #1 is located at 47999163 of chromosome #1, a CpG site of the sequence from 26394102 to 26396102 on chromosome #2 is located at 26395458 of chromosome #2, a CpG site of the sequence from 104510870 to 104513913 on chromosome #8 is located at 104512877 of chromosome #8, a CpG site of the sequence from 98289604 to 98290404 on chromosome #8 is located at 98290148 of chromosome #8, a CpG site of the sequence from 63281034 to 63281347 on chromosome #2 is located at 63281139 of chromosome #2, a CpG site of the sequence from 67873388 to 67875600 on chromosome #8 is located at 67874178 of chromosome #8, a CpG site of the sequence from 76555366 to 76556079 on chromosome #4 is located at 76555832 of chromosome #4, a CpG site of the sequence from 63782394 to 63790471 on chromosome #1 is located at 63789278 of chromosome #1, a CpG site of the sequence from 7849945 to 7850439 on chromosome #5 is located at 7850070 of chromosome #5, a CpG site of the sequence from 39186777 to 39187968 on chromosome #2 is located at 39187533 of chromosome #2, and a CpG site of the sequence from 74207665 to 74208665 on chromosome #14 is located at 74208165 of chromosome #14.

### [Item 5]

The method of item 1, wherein the biological sample is one selected from the group consisting of tissue, cells, blood, plasma, stool and urine derived from a patient with suspected liver cancer or a subject diagnosed with liver cancer.

### [Item 6]

The method of item 1, wherein the step (b) is performed by one method selected from the group consisting of PCR, methylation-specific PCR, real-time methylation-specific PCR, MethyLight PCR, MethyLight digital PCR, EpiTYPER, PCR using methylated DNA-specific binding protein, quantitative PCR, DNA chip assay, pyrosequencing and bisulfite sequencing.

### [Item 7]

The method of item 1, further comprising:
after step (b), (c) comparing the methylation level with a methylation level in a normal control.

### [Item 8]

A kit for diagnosing a risk of the onset of liver cancer, comprising:
a probe binding to a CpG site at a location selected from the group consisting of the sequence from 25438725 to 25439276 on chromosome #2, the sequence from 95941906 to 95942979 on chromosome #12, the sequence from 134597357 to 134602649 on chromosome #10, the sequence from 144649774 to 144651774 on chromosome #8, the sequence from 47998899 to 47999517 on chromosome #1, the sequence from 26394102 to 26396102 on chromosome #2, the sequence from 104510870 to 104513913 on chromosome #8, the sequence from 98289604 to 98290404 on chromosome #8, the sequence from 63281034 to 63281347 on chromosome #2, the sequence from 67873388 to 67875600 on chromosome #8, the sequence from 76555366 to 76556079 on chromosome #4, the sequence from 63782394 to 63790471 on chromosome #1, the sequence from 7849945 to 7850439 on chromosome #5, the sequence from 39186777 to 39187968 on chromosome #2, and the sequence from 74207665 to 74208665 on chromosome #14.

### [Item 9]

The kit of item 8, which comprises two or more probes binding to the CpG sites.

Hereinafter, the present invention will be described in further detail with reference to examples.

### Example 1. Selection of differentially methylated probe (DMP) associated with onset of liver cancer

### Samples

Liver cancer samples were obtained from 184 liver cancer patients in the Seoul National University Hospital to select a DNA methylation region associated with the onset of liver cancer. Normal tissue corresponding to liver cancer tissue was used as a normal control.

Genomic DNA was extracted from each sample using a column-based DNA extraction method (PureLink^{™} Genomic DNA Mini Kit, Invitrogen) and a bead-type DNA extraction method (MagListo^{™}5M Genomic DNA Extraction Kit, Bioneer). The extracted genomic DNA was quantified using NanoDrop, and a DNA state was identified by confirming degradation through electrophoresis in 1.5% agarose gel.

### Bisulfite treatment

The extent of methylation may be measured in such a way that, after genomic DNA is treated with bisulfite, when cytosine of the 5'-CpG-3' site of the DNA base sequence is methylated, the cytosine may remain the same, whereas when non-methylated, the cytosine is changed to uracil.

Therefore, to distinguish methylated cytosine and non-methylated cytosine, genomic DNA was treated with bisulfite. 700 ng of genomic DNA was treated using an EZ DNA Methylation Kit (Zymoresearch Inc.) according to the manufacturer's manual, and the bisulfite-treated DNA prepared thereby was dissolved in M-elution buffer and then stored at -80 °C before use.

The bisulfite-treated DNA was used within a month.

### DNA methylation microarray

A DNA methylation microarray was performed using Infinium^{®} Human Methylation 850K BeadChip.

Using Illumina Infinium Methylation EPIC BeadChip kits (Illumina, Inc., San Diego, CA) according to the manufacturer's manual, bisulfite-treated DNA was amplified, subjected to fragmentation, precipitation and resuspension, and hybridized with a BeadChip.

After washing, the BeadChip was scanned using an Illumina iScan scanner.

Among R packages, according to the manual in a package using a minfi package, the quality control of data was performed. Only for samples passing the standards of quality control, a β value, which is a value obtained by digitizing the idat file of raw data showing the extent of methylation in color, was calculated.

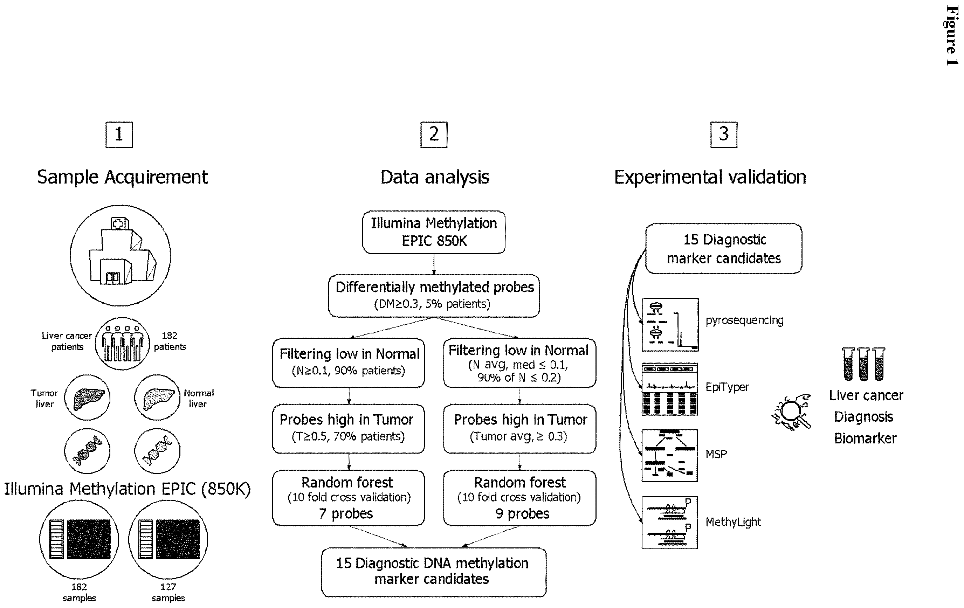

The extent of DNA methylation was represented as a β value of 0 to 1, where the β value of 0 represents that a corresponding CpG site is completely non-methylated, and the β value of 1 represents that a corresponding CpG site is completely methylated. The calculated results were normalized and corrected. All statistics were performed in the R statistical environment (v.3.3.2 or higher; FIG. 1).

### Example 2. Selection of diagnostic marker candidate

Referring to FIG. 1, DNA was extracted from 182 liver cancer samples and normal liver samples corresponding thereto, and subjected to an Infinium Methylation EPIC BeadChip assay.

Methylation data was analyzed with a self-constructed pipeline. Probes that exhibited low methylation levels in the normal samples and high methylation levels in the tumor samples were selected.

First, a DMP which had a methylation difference between a normal sample and a cancer sample was selected.

Seven probes which exhibited very low methylation levels in the normal samples and very high methylation levels of 50% or more in 70% or more of the cancer patients were selected, and efficiency was verified by a machine learning method (FIG. 1, dark blue).

Probes which exhibited very low methylation levels of 10% or less in the normal samples and high methylation levels of 30% or more on average in the liver cancer patients were selected, and the top nine probes that effectively distinguish liver cancer/normal liver samples were selected by machine learning (FIG. 1, brown).

The finally selected 15 (1 duplicate) liver cancer diagnostic marker candidates were verified by various experiments.

### Example 3. Selection of probes by heat map

As a result of investigating DNA methylation of 182 liver cancer samples and 127 normal samples, 100,053 DMP with 30% or more hypermethylation were selected from 5% or more of the liver cancer samples.

Among DMPs having a difference between normal/cancer samples, 13,078 probes exhibiting very low methylation levels of 10% or less were selected from normal samples such that blood biopsy was possible.

Among the selected probes, seven 50% or more-hypermethylated probes were selected from 70% or more of the cancer patients (Table 1).

**[Table 1]**

| **Division** | **Probe ID** | **50% or more hypermethylated liver cancer ratio (%)** |
|---|---|---|
| Probe 1 | cg20172627 | 78.16 |
| Probe 2 | cg22538054 | 77.59 |
| Probe 3 | cg27583690 | 74.14 |
| Probe 4 | cg19951303 | 72.99 |
| Probe 5 | cg22524657 | 71.84 |
| Probe 6 | cg24563094 | 70.11 |
| Probe 7 | cg25744484 | 70.11 |

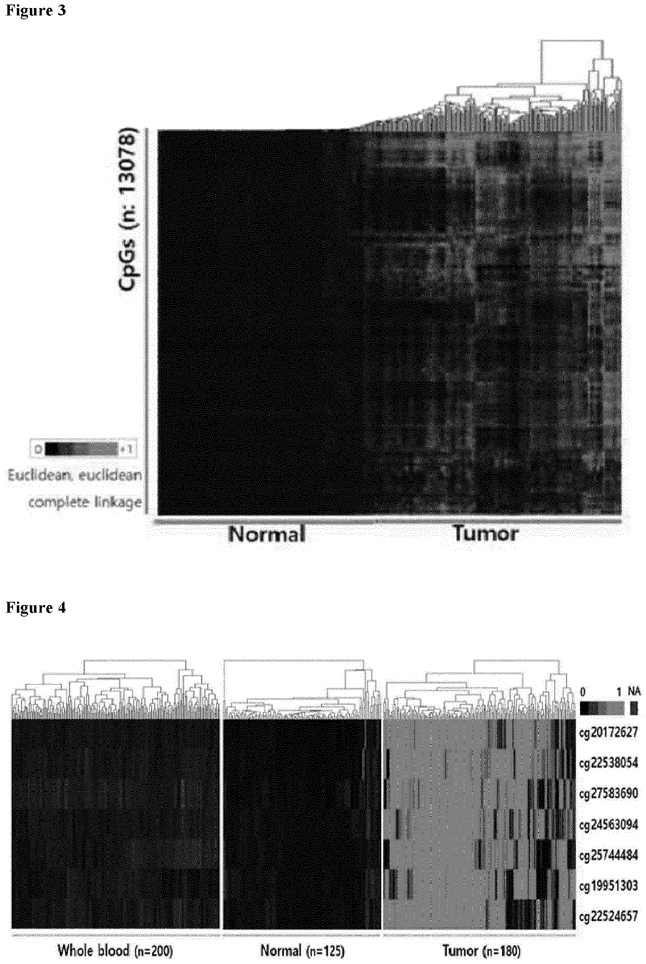

Heat maps were made to confirm liver cancer patient-specific methylation levels of the selected seven probes (FIG. 4).

### Example 4. Selection of probe by machine learning

Among DMPs showing a difference between normal/cancer samples, probes that exhibit very low methylation levels in normal samples and high methylation levels of 30% or more on average in liver cancer patients were selected.

The top nine probes effectively distinguishing liver cancer/normal liver samples were selected through machine learning using the previously-selected probes.

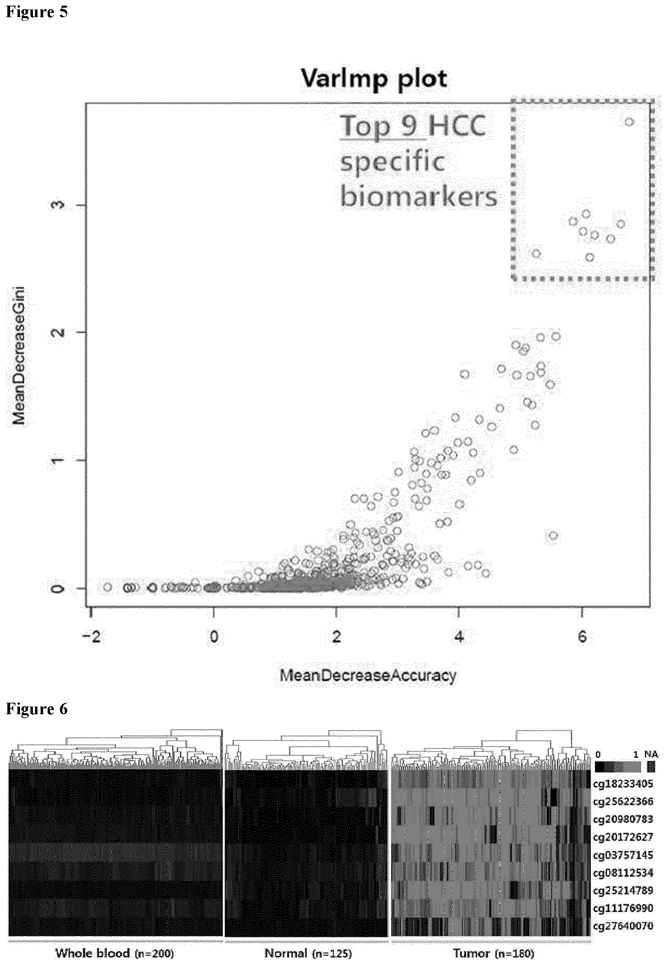

Referring to FIG. 5, a blue dot represents one probe, and the top nine probes were selected in order of importance (x- and y-axes).

The x-axis represents the accuracy of each probe in a model constructed by machine learning, and the y-axis represents the purity of each probe in a model constructed by machine learning.

Heat maps were made to confirm the extent of methylation of the 9 probes selected by machine learning in 200 whole blood samples, 125 normal samples and 180 liver cancer samples (FIG. 6).

Information on the 15 probes finally selected by the methods described in Examples 4 and 5 is shown in Table 2 below.

**[Table 2]**

| **SEQ ID NO:** | **Probe ID** | **Selection method** | **CpG location** | | | **CGI region** |
|---|---|---|---|---|---|---|
| | | | **Chromosome** | **start** | **End** | |
| 1 | cg20172627 | heatmap | chr2 | 25438725 | 25439276 | Island |
| 2 | cg22538054 | heatmap | chr12 | 95941906 | 95942979 | Island |
| 3 | cg27583690 | heatmap | chr10 | 134597357 | 134602649 | Island |
| 4 | cg19951303 | heatmap | chr8 | 144649774 | 144651774 | N_Shelf |
| 5 | cg22524657 | heatmap | chr1 | 47998899 | 47999517 | Island |
| 6 | cg24563094 | heatmap | chr2 | 26394102 | 26396102 | N_Shore |
| 7 | cg25744484 | heatmap | chr8 | 104510870 | 104513913 | Island |
| 8 | cg18233405 | machine learning | chr8 | 98289604 | 98290404 | Island |
| 9 | cg25622366 | machine learning | chr2 | 63281034 | 63281347 | Island |
| 10 | cg20980783 | machine learning | chr8 | 67873388 | 67875600 | Island |
| 1 | cg20172627 | machine learning | chr2 | 25438725 | 25439276 | Island |
| 11 | cg03757145 | machine learning | chr4 | 76555366 | 76556079 | Island |
| 12 | cg08112534 | machine learning | chr1 | 63782394 | 63790471 | Island |
| 13 | cg25214789 | machine learning | chr5 | 7849945 | 7850439 | Island |
| 14 | cg11176990 | machine learning | chr2 | 39186777 | 39187968 | Island |
| 15 | cg27640070 | machine learning | chr14 | 74207665 | 74208665 | - |

### Example 5. Evaluation of liver cancer diagnosis efficiency of single probes

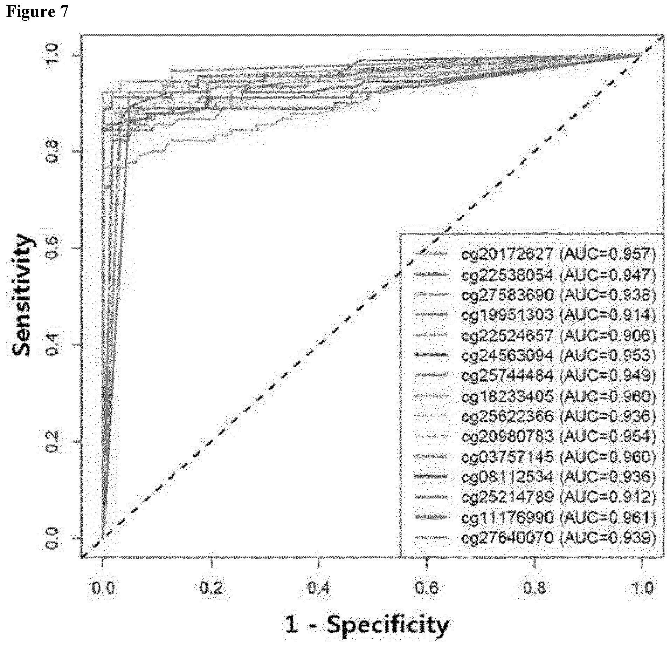

The liver cancer diagnostic efficiency of the selected 15 probes was evaluated (FIG. 7).

FIG. 7 shows the liver cancer diagnostic efficiency per probe, represented as AUC.

Liver cancer diagnostic efficiency (AUC; area under the curve) was confirmed only using 15 probes, and the results are shown in Table 3 below.

**[Table 3]**

| **SEQ ID NO:** | **probe ID** | **Selection method** | **Acuu.** | **Sen.** | **Spe.** | **AUC** |
|---|---|---|---|---|---|---|
| 1 | cg20172627 | heatmap | 0.908 | 0.922 | 0.887 | 0.957 |
| 2 | cg22538054 | heatmap | 0.888 | 0.878 | 0.903 | 0.947 |
| 3 | cg27583690 | heatmap | 0.863 | 0.856 | 0.873 | 0.938 |
| 4 | cg19951303 | heatmap | 0.837 | 0.889 | 0.762 | 0.914 |
| 5 | cg22524657 | heatmap | 0.811 | 0.822 | 0.794 | 0.906 |
| 6 | cg24563094 | heatmap | 0.889 | 0.922 | 0.841 | 0.953 |
| 7 | cg25744484 | heatmap | 0.882 | 0.889 | 0.871 | 0.949 |
| 8 | cg18233405 | machine learning | 0.948 | 0.944 | 0.952 | 0.960 |
| 9 | cg25622366 | machine learning | 0.908 | 0.889 | 0.936 | 0.936 |
| 10 | cg20980783 | machine learning | 0.888 | 0.878 | 0.903 | 0.954 |
| 11 | cg03757145 | machine learning | 0.909 | 0.922 | 0.889 | 0.960 |
| 12 | cg08112534 | machine learning | 0.855 | 0.889 | 0.807 | 0.936 |
| 13 | cg25214789 | machine learning | 0.863 | 0.889 | 0.825 | 0.912 |
| 14 | cg11176990 | machine learning | 0.882 | 0.922 | 0.823 | 0.961 |
| 15 | cg27640070 | machine learning | 0.895 | 0.900 | 0.889 | 0.939 |

In addition, from the public DB, the liver cancer diagnostic efficiency of a single probe was verified (FIG. 7). FIG. 7 shows the liver cancer diagnostic efficiency per probe, represented as AUC.

The result of verifying the efficiency of single probes using TCGA LIHC methylation data (450K) is shown in Table 4 below.

The region marked with gray(-) indicates a probe which is not found in an Infinium Methylation 450K BeadChip, and found only in an Infinium Methylation EPIC BeadChip (850K).

**[Table 4]**

| **SEQ ID NO:** | **Probe ID** | **Selection method** | **Acuu.** | **Sen.** | **Spe.** | **AUC** |
|---|---|---|---|---|---|---|
| 1 | cg20172627 | heatmap | 0.916 | 0.918 | 0.900 | 0.957 |
| 2 | cg22538054 | heatmap | 0.797 | 0.786 | 0.880 | 0.897 |
| 3 | cg27583690 | heatmap | 0.764 | 0.754 | 0.840 | 0.855 |
| 4 | cg19951303 | heatmap | - | - | - | - |
| 5 | cg22524657 | heatmap | 0.816 | 0.815 | 0.820 | 0.902 |
| 6 | cg24563094 | heatmap | 0.870 | 0.876 | 0.820 | 0.919 |
| 7 | cg25744484 | heatmap | - | - | - | - |
| 8 | cg18233405 | machine learning | 0.893 | 0.902 | 0.820 | 0.919 |
| 9 | cg25622366 | machine learning | 0.888 | 0.879 | 0.960 | 0.967 |
| 10 | cg20980783 | machine learning | 0.897 | 0.897 | 0.900 | 0.935 |
| 11 | cg03757145 | machine learning | 0.890 | 0.879 | 0.980 | 0.939 |
| 12 | cg08112534 | machine learning | - | - | - | - |
| 13 | cg25214789 | machine learning | 0.881 | 0.887 | 0.840 | 0.916 |
| 14 | cg11176990 | machine learning | 0.846 | 0.852 | 0.800 | 0.933 |
| 15 | cg27640070 | machine learning | - | - | - | - |

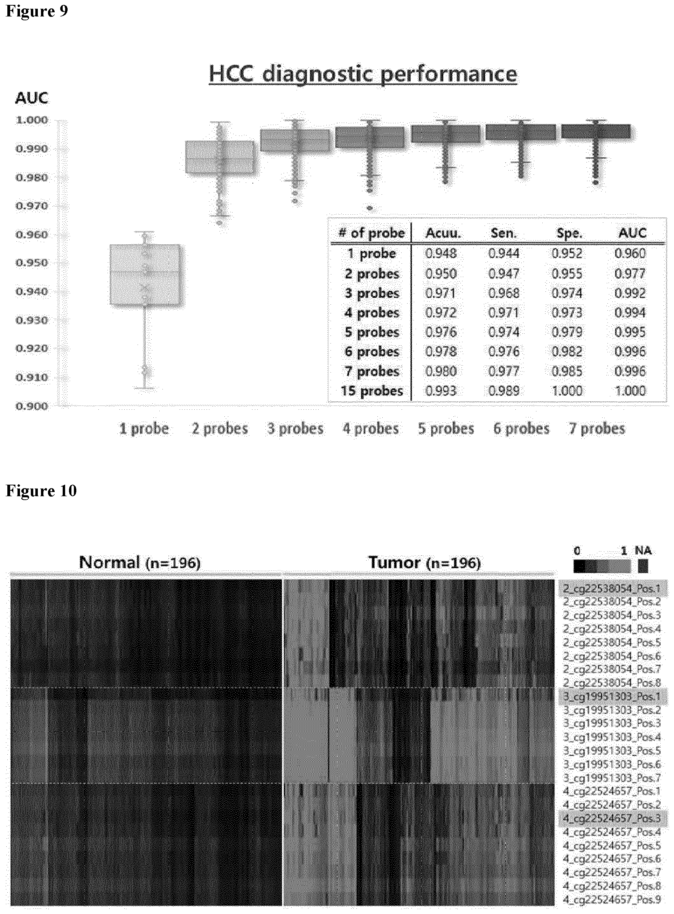

In addition, to analyze the liver cancer diagnostic efficiency of 15 panel probes, the liver cancer diagnostic efficiency (AUC) was confirmed by combining 15 probes (FIG. 9). FIG. 9 shows the confusion matrix result of training data and validation data obtained by machine learning with 15 probes (secondary cross validation).

To prevent data bias, secondary cross validation for randomly dividing data into two sets was performed 10 times, and thus the data was classified into a testing set and a training set.

Based on the data classified as the training set, normal and liver cancer patterns were learned, and a liver cancer-specific diagnosis model according thereto was constructed.

Table 5 shows an error matrix of the training set.

**[Table 5]**

| Input value | Determined as normal | Determined as liver cancer | Error rate |
|---|---|---|---|
| Normal | 62 | 1 | 0.159 |
| Liver cancer | 3 | 87 | 0.333 |

The test set was diagnosed based on the liver cancer-specific diagnosis model constructed with the training set, thereby confirming liver cancer diagnostic efficiency (Table 6).

**[Table 6]**

| Sample | Determined as normal | Determined as liver cancer |
|---|---|---|
| Normal | 61 | 0 |
| Liver cancer | 1 | 90 |

Referring to Tables 5 and 6, a liver cancer-specific diagnosis model was able to be constructed with the 15 probes selected based on machine learning, and diagnostic efficiency was evaluated at a very high level.

### Example 6. Evaluation of liver cancer diagnosis efficiency using several probes

To determine the minimum number of probes having the maximum efficiency among the 15 probes based on the liver cancer-specific diagnosis model, efficiency per the number of probes was measured (FIG. 9).

FIG. 9 shows the result obtained by machine learning with possible probe combinations (secondary cross validation). The x-axis represents the number of probes, and the y-axis represents AUC (diagnostic efficiency).

Referring to FIG. 9, when the number of probes is 3 or more, since the diagnostic efficiency approaches 99% or more, very accurate diagnosis data may be provided.

Accordingly, compared with the use of a single probe, the use of several probes may significantly improve diagnostic accuracy.

### Example 7. Analysis of methylation of CpG island including probe by pyrosequencing

To measure the extent of methylation at a CpG site to which one of the selected probes is bound, pyrosequencing was performed.

Pyrosequencing uses a pyrophosphate (PPi) emitted by addition of a nucleotide. PPi is converted into ATP by ATP sulfurylase in the presence of adenosine 5'-phosphate.

Luciferase is used to convert luciferin into oxyluciferin by ATP, and this reaction produces light that is able to be detected and analyzed.

The extent of methylation at CpG sites of the selected probes are shown by heat maps (FIG. 10).

As a result, it was confirmed that the methylation level was low in Normal, and high in Tumor, and the extent of methylation at CpG sites of the selected probes and their surroundings was similar.

### Example 8. Analysis of methylation of CpG island including probe by EpiTYPER

To verify data, methylation states of the top three probes among the selected probes were quantitatively analyzed using an EpiTYPER^{™} assay (Sequenom, San Diego, CA).

After PCR amplification, amplicons transcribed *in vitro* were treated with shrimp alkaline phosphatase, cleaved with RNaseA, and to determine the methylation state, subjected to MALDI-TOF Mass Spectrometry.

The result was analyzed using EpiTYPER^{™} ver. 1.0 software.

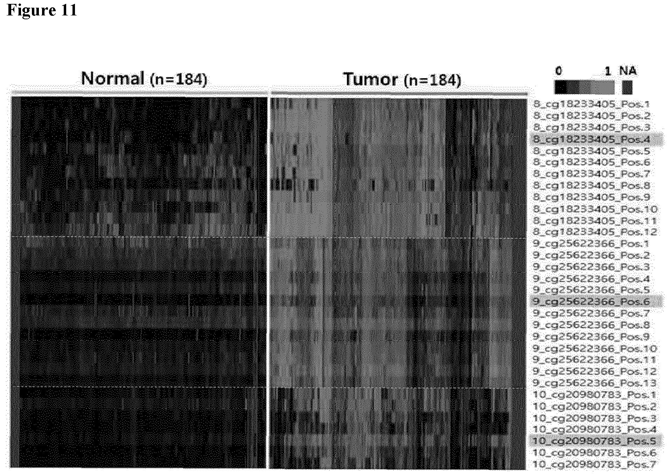

Validation was performed for the three selected probes by EpiTYPER. The extent of methylation at CpG sites of the selected probes and their surroundings was confirmed by heat maps (FIG. 11).

Referring to FIG. 11, it was confirmed that the methylation level was low in Normal and high in Tumor, and the extent of methylation at CpG sites of the selected probes and their surroundings was similar.

Accordingly, methylation levels of all of the CpG islands including a CPG probe can be used for diagnosing the prognosis and risk of cancer as described above.

It should be understood by those of ordinary skill in the art that the above description of the present invention are exemplary, and the example embodiments disclosed herein can be easily modified into other specific forms without changing the technical spirit or essential features of the present invention. Therefore, it should be interpreted that the example embodiments described above are exemplary in all aspects, and are not limitative. For example, each component described as a single unit may be distributed and implemented, and components described as being distributed may also be implemented in combined form.

The scope of the present invention is defined by the appended claims and encompasses all modifications and alterations derived from meanings, the scope and equivalents of the appended claims.

## Claims

1. An *in vitro* method of diagnosing liver cancer, said method comprising the step of: measuring a methylation level of a CpG site in the sequence from 47998899 to 47999517 on chromosome #1 in DNA isolated from a biological sample of a subject.

2. The method of claim 1, wherein said diagnosing liver cancer comprises determining whether a subject currently has liver cancer.

3. The method of claim 1 or 2, wherein said method further comprises measuring a methylation level of a CpG site at least one further location selected from the group consisting of: the sequence from 98289604 to 98290404 on chromosome #8, the sequence from 25438725 to 25439276 on chromosome #2, the sequence from 95941906 to 95942979 on chromosome #12, the sequence from 134597357 to 134602649 on chromosome #10, the sequence from 144649774 to 144651774 on chromosome #8, the sequence from 26394102 to 26396102 on chromosome #2, the sequence from 104510870 to 104513913 on chromosome #8, the sequence from 63281034 to 63281347 on chromosome #2, the sequence from 67873388 to 67875600 on chromosome #8, the sequence from 76555366 to 76556079 on chromosome #4, the sequence from 63782394 to 63790471 on chromosome #1, the sequence from 7849945 to 7850439 on chromosome #5, the sequence from 39186777 to 39187968 on chromosome #2, and the sequence from 74207665 to 74208665 on chromosome #14 in the isolated DNA.

4. The method of any one of claims 1 to 3, wherein the sequence from 47998899 to 47999517 on chromosome #1 has the base sequence of SEQ ID NO: 5.

5. The method of any one of claims 1 to 4, wherein the CpG site in the sequence from 47998899 to 47999517 on chromosome #1 is located at 47999163 of chromosome #1.

6. The method of any one of claims 3 to 5, wherein the sequence from 98289604 to 98290404 on chromosome #8 has the base sequence of SEQ ID NO: 8, wherein the sequence from 25438725 to 25439276 on chromosome #2 has the base sequence of SEQ ID NO: 1, the sequence from 95941906 to 95942979 on chromosome #12 has the base sequence of SEQ ID NO: 2, the sequence from 134597357 to 134602649 on chromosome #10 has the base sequence of SEQ ID NO: 3, the sequence from 144649774 to 144651774 on chromosome #8 has the base sequence of SEQ ID NO: 4, the sequence from 26394102 to 26396102 on chromosome #2 has the base sequence of SEQ ID NO: 6, the sequence from 104510870 to 104513913 on chromosome #8 has the base sequence of SEQ ID NO: 7, the sequence from 63281034 to 63281347 on chromosome #2 has the base sequence of SEQ ID NO: 9, the sequence from 67873388 to 67875600 on chromosome #8 has the base sequence of SEQ ID NO: 10, the sequence from 76555366 to 76556079 on chromosome #4 has the base sequence of SEQ ID NO: 11, the sequence from 63782394 to 63790471 on chromosome #1 has the base sequence of SEQ ID NO: 12, the sequence from 7849945 to 7850439 on chromosome #5 has the base sequence of SEQ ID NO: 13, the sequence from 39186777 to 39187968 on chromosome #2 has the base sequence of SEQ ID NO: 14, and/or the sequence from 74207665 to 74208665 on chromosome #14 has the base sequence of SEQ ID NO: 15.

7. The method of any one of claims 3 to 6, wherein the CpG site in the sequence from 98289604 to 98290404 on chromosome #8 is located at 98290148 of chromosome #8, wherein the CpG site in the sequence from 25438725 to 25439276 on chromosome #2 is located at 25439110 of chromosome #2, the CpG site in the sequence from 95941906 to 95942979 on chromosome #12 is located at 95941988 of chromosome #12, the CpG site in the sequence from 134597357 to 134602649 on chromosome #10 is located at 134599823 of chromosome #10, the CpG site in the sequence from 144649774 to 144651774 on chromosome #8 is located at 144651002 of chromosome #8, the CpG site in the sequence from 26394102 to 26396102 on chromosome #2 is located at 26395458 of chromosome #2, the CpG site in the sequence from 104510870 to 104513913 on chromosome #8 is located at 104512877 of chromosome #8, the CpG site in the sequence from 63281034 to 63281347 on chromosome #2 is located at 63281139 of chromosome #2, the CpG site in the sequence from 67873388 to 67875600 on chromosome #8 is located at 67874178 of chromosome #8, the CpG site in the sequence from 76555366 to 76556079 on chromosome #4 is located at 76555832 of chromosome #4, the CpG site in the sequence from 63782394 to 63790471 on chromosome #1 is located at 63789278 of chromosome #1, the CpG site in the sequence from 7849945 to 7850439 on chromosome #5 is located at 7850070 of chromosome #5, the CpG site in the sequence from 39186777 to 39187968 on chromosome #2 is located at 39187533 of chromosome #2, and/or the CpG site in the sequence from 74207665 to 74208665 on chromosome #14 is located at 74208165 of chromosome #14.

8. The method of any one of claims 1 to 7, wherein the biological sample is selected from the group consisting of tissue, cells, blood, plasma, stool and urine derived from a patient with suspected liver cancer or a subject diagnosed with liver cancer.

9. The method of any one of claims 1 to 8, wherein said measurement of the methylation level is performed by a method selected from the group consisting of PCR, methylation-specific PCR, real-time methylation-specific PCR, MethyLight PCR, MethyLight digital PCR, EpiTYPER, PCR using methylated DNA-specific binding protein, quantitative PCR, DNA chip assay, pyrosequencing and bisulfite sequencing.

10. The method of any one of claims 1 to 9, further comprising as a following step: comparing the measured methylation level with a methylation level in a normal control.

11. A kit for diagnosing liver cancer comprising a set of probes, wherein said set of probes consists of one or more probes binding to a CpG site at a location selected from the group consisting of the sequence from 25438725 to 25439276 on chromosome #2, the sequence from 95941906 to 95942979 on chromosome #12, the sequence from 134597357 to 134602649 on chromosome #10, the sequence from 144649774 to 144651774 on chromosome #8, the sequence from 47998899 to 47999517 on chromosome #1, the sequence from 26394102 to 26396102 on chromosome #2, the sequence from 104510870 to 104513913 on chromosome #8, the sequence from 98289604 to 98290404 on chromosome #8, the sequence from 63281034 to 63281347 on chromosome #2, the sequence from 67873388 to 67875600 on chromosome #8, the sequence from 76555366 to 76556079 on chromosome #4, the sequence from 63782394 to 63790471 on chromosome #1, the sequence from 7849945 to 7850439 on chromosome #5, the sequence from 39186777 to 39187968 on chromosome #2, and the sequence from 74207665 to 74208665 on chromosome #14, and wherein said set of probes comprises at least a probe binding to a CpG site in the sequence from 47998899 to 47999517 on chromosome #1.

12. The kit of claim 11, which comprises at least one further probe binding to a CpG site at a location selected from the group consisting of the sequence from 98289604 to 98290404 on chromosome #8, the sequence from 25438725 to 25439276 on chromosome #2, the sequence from 95941906 to 95942979 on chromosome #12, the sequence from 134597357 to 134602649 on chromosome #10, the sequence from 144649774 to 144651774 on chromosome #8, the sequence from 26394102 to 26396102 on chromosome #2, the sequence from 104510870 to 104513913 on chromosome #8, the sequence from 63281034 to 63281347 on chromosome #2, the sequence from 67873388 to 67875600 on chromosome #8, the sequence from 76555366 to 76556079 on chromosome #4, the sequence from 63782394 to 63790471 on chromosome #1, the sequence from 7849945 to 7850439 on chromosome #5, the sequence from 39186777 to 39187968 on chromosome #2, and the sequence from 74207665 to 74208665 on chromosome #14.

13. A kit for use in diagnosing liver cancer, comprising a probe binding to a CpG site in the sequence from 47998899 to 47999517 on chromosome #1 in DNA isolated from a biological sample of a subject.

14. Use of a probe for preparing a kit for diagnosing liver cancer, wherein the probe binds to a CpG site in the sequence from 47998899 to 47999517 on chromosome #1 in DNA isolated from a biological sample of a subject.
